(19) Europäisches Patentamt
European Patent Office
Office européen des brevets

(11) EP 3 817 824 B1

(12) **EUROPEAN PATENT SPECIFICATION**

(45) Date of publication and mention of the grant of the patent:
**25.10.2023 Bulletin 2023/43**

(21) Application number: **19732677.0**

(22) Date of filing: **26.06.2019**

(51) International Patent Classification (IPC):
***A61Q 1/00*** (2006.01) ***A61Q 1/02*** (2006.01)
***A61Q 1/08*** (2006.01) ***A61Q 1/10*** (2006.01)
***A61K 8/19*** (2006.01) ***A61K 8/29*** (2006.01)
***A61K 8/31*** (2006.01) ***A61K 8/34*** (2006.01)
***A61K 8/88*** (2006.01)

(52) Cooperative Patent Classification (CPC):
**A61Q 1/00; A61K 8/19; A61K 8/29; A61K 8/31; A61K 8/34; A61K 8/88; A61Q 1/02; A61Q 1/08; A61Q 1/10;** A61K 2800/31

(86) International application number:
**PCT/EP2019/067054**

(87) International publication number:
**WO 2020/007686 (09.01.2020 Gazette 2020/02)**

(54) **FLUID COMPOSITION COMPRISING AN ESTER-TERMINATED POLY(ESTER-AMIDE) POLYMER, A VOLATILE ALKANE, A MONOALCOHOL AND AT LEAST ONE PIGMENT**

FLÜSSIGKEITSZUSAMMENSETZUNG MIT EINEM ESTERTERMINIERTEN POLY(ESTERAMID)POLYMER, EINEM FLÜCHTIGEN ALKAN, EINEM MONOALKOHOL UND MINDESTENS EINEM PIGMENT

COMPOSITION FLUIDE COMPRENANT UN POLYMÈRE POLY(ESTER-AMIDE) À TERMINAISON ESTER, UN ALCANE VOLATIL, UN MONOALCOOL ET AU MOINS UN PIGMENT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **02.07.2018 FR 1856081**

(43) Date of publication of application:
**12.05.2021 Bulletin 2021/19**

(73) Proprietor: **L'OREAL**
**75008 Paris (FR)**

(72) Inventor: **KERGOSIEN, Guillaume**
**94152 CHEVILLY LARUE (FR)**

(74) Representative: **L'Oreal**
**Service D.I.P.I.**
**9, rue Pierre Dreyfus**
**92110 Clichy (FR)**

(56) References cited:
**EP-A1- 1 813 266**
**WO-A1-2009/071675**
**WO-A1-2018/115771**
**US-A1- 2005 197 479**
**US-A1- 2008 233 065**
**US-A1- 2011 150 799**
**US-B2- 6 552 160**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Processed by Luminess, 75001 PARIS (FR)

## Description

The present invention aims to provide, for the field of caring for and/or making up keratin materials, in particular the skin, a new composition comprising at least one ester-terminated poly(ester-amide) polymer, at least one volatile alkane, at least one alcohol and a particulate phase comprising at least one pigment.

Cosmetic makeup compositions are commonly used to give keratin materials such as the skin an aesthetic colour, but also to enhance the beauty of irregular skin, by hiding marks and dyschromias, and to reduce the visibility of relief imperfections such as pores and wrinkles. Many formulations have been developed to date. For a few years, consumers have been searching for compositions which provide a good wear property of the make-up effect over time in order to be able to avoid having to re-apply the composition too often.

Consumers increasingly look for cosmetic makeup products which spread easily and rapidly, in particular on the skin in the form of a deposit which must not be thick but, on the contrary, must disappear as much as possible into the support for coverage with neither marking nor a mask effect. They are thus looking for makeup products with fluid, lighter presentation forms which are easy to apply and with good wear properties and comfort properties (mattness persistence, colour persistence, homogeneity of the deposit, non-oily, non-tacky appearance).

One of the solutions proposed by the prior art for obtaining a good wear property is the use of a film-forming agent which allows the composition, once applied, to form, after drying, a more cohesive, adhesive and persistent film on the support. However, the use of film-forming agents is not entirely satisfactory since one of the problems encountered with such agents lies in the fact that some cause discomfort, through for example a tacky effect and/or difficulty in spreading the composition. Others have a tendency to thicken or even gel the product.

US document 2005/0197479 proposes the use of specific gelling agents in solid cosmetic compositions (sticks), such as deodorants or lipsticks in gel form or high viscosity formulations such as shampoos.

WO 2018/115771 pertains to a solid anhydrous cosmetic composition such as an eye shadow, a make-up foundation or a lipstick comprising an ester-terminated polyamide resin, a silicate and an oil.

In point of fact, the applicant has now discovered, surprisingly, that this objective can be achieved with an anhydrous fluid composition comprising, in a cosmetically acceptable medium:

a) at least one ester-terminated poly(ester-amide) polymer;
b) at least one volatile alkane and
c) at least one monoalcohol comprising from 2 to 8 carbon atoms and
d) a particulate phase comprising at least one pigment.

The applicant has in fact noted, during its research, that the fluid composition according to the invention results in compositions which have an excellent wear property and are easy to apply to the skin.

The invention also relates to a process for coating keratin materials, more particularly for making up and/or caring for keratin materials, such as the skin, characterized in that it comprises the application to the keratin materials of a composition as defined previously.

The present invention also relates to a cosmetic assembly comprising

- an anhydrous fluid composition comprising, in a cosmetically acceptable medium:

  a) at least one ester-terminated poly(ester-amide) polymer;
  b) at least one volatile alkane and
  c) at least one monoalcohol comprising from 2 to 8 carbon atoms and
  d) a particulate phase comprising at least one pigment

- a device for applying said composition to the keratin materials.

This discovery forms the basis of the present invention.

Other characteristics, aspects and advantages of the invention will become apparent on reading the detailed description that follows.

## DEFINITIONS

In the context of the present invention, the term "keratin material" is intended to mean in particular the skin (of the body, face, around the eyes, or the eyelids).

The term "physiologically acceptable" is intended to mean compatible with the skin and/or its integuments, which has a pleasant colour, odour and feel, and which does not cause any unacceptable discomfort (stinging or tautness)

liable to discourage the consumer from using this composition.

The term "fluid" is understood to mean a liquid composition which flows under its own weight at ambient temperature (25°C) and atmospheric pressure.

Advantageously, a fluid composition according to the invention has a complex modulus of rigidity G* of less than 1000 Pa, preferably less than 400 Pa, preferably less than 200 Pa.

These are harmonic-regime rheological measurements for measuring the elastic modulus.

The measurements are carried out using a Haake RS600 rheometer on a product at rest, at 25°C.

The harmonic-regime measurements make it possible to characterize the viscoelastic properties of the products. The technique consists of subjecting a material to a stress which varies sinusoidally over time and in measuring the response of the material to this stress. In a range in which the behaviour is linear viscoelastic behaviour (zone in which the strain is proportional to the stress), the stress ($\tau$) and the strain ($\gamma$) are two sinusoidal functions of time which are written in the following manner:

$$\tau(t) = \tau 0 \sin(\omega t)$$

$$\gamma(t) = \gamma 0 \sin(\omega t + \delta)$$

in which:

$\tau 0$ represents the maximum amplitude of the stress (Pa);
$\gamma 0$ represents the maximum amplitude of the strain (-);
$\omega = 2\Pi N$ represents the angular frequency (rad.s-1) with N representing the frequency (Hz); and
$\delta$ represents the phase shift of the stress relative to the strain (rad).

Thus, the two functions have the same angular frequency but they are shifted by an angle $\delta$. According to the phase shift $\delta$ between $\tau(t)$ and $\gamma(t)$, the behaviour of the system may be apprehended:

- if $\delta = 0$, the material is purely elastic;
- if $\delta = \Pi/2$, the material is purely viscous (Newtonian fluid); and
- if $0 < \delta < \Pi/2$, the material is viscoelastic.

In general, the stress and the strain are written in complex form:

$$\tau^*(t) = \tau 0\ ei\omega t$$

$$\gamma^*(t) = \gamma 0\ e(i\omega t + \delta)$$

A complex modulus of rigidity, representing the overall resistance of the material to strain, whether it is of elastic or viscous origin, is then defined by:

$$G^* = \tau^*/\gamma^* = G' + iG''$$

In which:

G' is the storage modulus or elastic modulus which characterizes the energy stored and totally restored during a cycle, $G' = (\tau 0/\gamma 0) \cos \delta$; and
G" is the loss modulus or viscous modulus which characterizes the energy dissipated by internal friction during a cycle, $G'' = (\tau 0/\gamma 0) \sin \delta$.

The parameter retained is the mean modulus of rigidity G* recorded at the plateau measured at a frequency of 1 Hz.

**ESTER-TERMINATED POLY(ESTER-AMIDE)**

The ester-terminated poly(ester-amide) (ETPEA) polymer in accordance with the invention is preferably in the form of a resin prepared by reacting a diacid, a diamine, a polyol and a monoalcohol in which:

(i) at least 50 equivalent % of said diacid comprises a polymerized fatty acid and
(ii) at least 50 equivalent % of said diamine comprises ethylenediamine.

More preferentially, the resin composition is such that

(iii) 10 to 60 equivalent % relative to all of the equivalents of hydroxyl and of amine originating from the diamine, from the polyol and from the monoalcohol originate from the monoalcohol,
(iv) at most 50 equivalent % relative to all of the equivalents of hydroxyl and of amine originating from the diamine, from the polyol and from the monoalcohol originate from the polyol.

The ester-terminated poly(ester-amide) (ETPEA) polymers in accordance with the invention can be prepared according to the process described in patent US 6 552 160.
The diacid is in general an organic molecule containing two carboxylic acid groups or equivalent reactive groups. Preferentially, the diacid is a polymerized fatty acid.
The polymerized fatty acid is typically a mixture comprising an acid dimer and an acid trimer where each dimer may be saturated, unsaturated, cyclic or acyclic.
The polymerized fatty acid used for the synthesis of the ester-terminated poly(ester-amide) (ETPEA) polymer is preferably an acid dimer.
The polymerized fatty acid is in general formed by heating long-chain unsaturated fatty acids, for example $C_{18}$ monocarboxylic acids, at temperatures of about 200-250°C in the presence of a catalyst clay in order to polymerize the fatty acids. The product obtained comprises in general an acid dimer, in particular a $C_{36}$ dicarboxylic acid formed by dimerization of the fatty acid and an acid trimer, in particular a $C_{54}$ tricarboxylic acid obtained by trimerization of the carboxylic acid. Greater details regarding the polymerization of fatty acids are indicated in particular in patent US 3 157 681 and the book "Naval Stores--Production, Chemistry and Utilization, D.F. Zinkel and J. Russell (eds.), Pulp. Chem. Assoc. Inc., 1989, Chapter 23".
Preferentially, the polymerized fatty acid contains less than 20% by weight of acid trimer and at least 80% by weight of acid dimer relative to the total weight of the polymerized fatty acid. More particularly, the acid dimer constitutes essentially all of the polymerized fatty acid.
Among the unsaturated fatty acids used to form the polymerized fatty acid, mention may be made of oleic acid, linoleic acid and linolenic acid. Use is preferably made of long-chain fatty acid oils which are mixtures of long-chain unsaturated acids obtained by means of a wood-pulp reduction process. Use may also be made of other sources, such as soybeans or canola seeds. The polymerized fatty acid that can be used according to the invention has an acid number of about 180 to 200.
The polymerized fatty acid may be hydrogenated before being used in the resin formation reaction. Hydrogenation makes it possible to obtain a slightly higher melting point of the resin and also a greater stability with respect to oxidation and of the colour in the case of a slightly coloured resin.
Among the polymerized fatty acids and in particular the hydrogenated forms that are commercially available, mention may be made of the product sold under the brand Unidyme by the company Arizona Chemical, the product sold under the brand Pripol 1015 by the company Uniqema, or the product sold under the brand Empol 1008 by the company Cognis.
Use will more particularly be made, as polymerized fatty acid, of a $C_{36}$ hydrogenated linoleic acid dimer.
In addition to the polymerized fatty acid or reactive equivalents, the diacid may comprise a co-diacid of formula: $HOOC-R_1-COOH$ where $R_1$ is a $C_4$-$C_{19}$, preferably $C_4$-$C_{12}$ and more preferentially $C_4$-$C_8$ hydrocarbon-based compound.
The carbon atoms may be arranged in linear, branched or cyclic form and an unsaturation may be present between two adjacent atoms. $R_1$ may be aliphatic or aromatic.
The diamine reagent possesses two amine groups which are preferably primary amines and represented by the formula: $HN(R_{2a})--R_2--N(R_{2a})H$ in which $R_2$a denotes hydrogen or an alkyl group or else forms, with $R_2$ or another radical $R_{2a}$, a heterocycle.
As diamine, use will more particularly be made of ethylenediamine, i.e.: $R_{2a}$ is hydrogen and $R_2$ is $--CH_2--\ CH_2--$.
The diamines other than ethylenediamine will be denoted in the text as co-diamines. When they are present, the co-diamines are used in low amounts relative to the ethylenediamine.
The monoalcohol may be represented by the formula $R_3$-OH where $R_3$ is preferably a hydrocarbon-based group having at least 10 carbon atoms. Thus, the monoalcohol may be described as a monohydric alcohol.

According to one particular form, $R_3$ is a $C_{10}$-$C_{30}$ hydrocarbon-based group, preferentially a $C_{12}$-$C_{24}$ hydrocarbon-based group and even more particularly a $C_{18}$ hydrocarbon-based radical. For the purposes of the invention, the term "$C_{10}$-$C_{30}$ hydrocarbon-based group" is intended to mean any group having at least 10 carbon atoms but at most 30 carbon atoms. The carbon atoms may be arranged in a linear, branched or cyclic manner and the hydrocarbon-based radical may be saturated or unsaturated.

According to one particularly preferred form, $R_3$ is linear and the hydroxyl group is located on an end carbon: i.e. the monoalcohol is primary. Among the monoalcohols that can be used to prepare the ETPEA resin, mention may be made of 1-dodecanol, 1-tetradecanol, 1-hexadecanol (cetyl alcohol), 1-octadecanol (stearyl alcohol), 1-eicosanol (arachidyl alcohol) and 1-docosanol (behenyl alcohol).

The reactive monoalcohol may contain an alkylene group, i.e. an alkyl group with an unsaturation between two adjacent carbon atoms.

Another reactive monoalcohol that can be used according to the invention may be a Guerbet alcohol of formula: H--C($R_a$)($R_b$)--$CH_2$--OH where $R_a$ and $R_b$, which may be identical or different, preferably denote a $C_6$-$C_{12}$ hydrocarbon-based group. Guerbet alcohols are in particular described in the book "Dictionary For Auxiliaries For Pharmacy, Cosmetics And Related Fields," H. P. Fiedler, 3rd Ed., 1989, Cantor Aulendorf. Hexadecyloctadecanol-2 having 24 carbon atoms will be more particularly used.

Another type of reactive monoalcohol that can be used according to the invention is a linear alcoholic wax. Among the linear alcoholic waxes available on the market, mention may be made of the products sold under the brand Unilin by the company Petrolite Corporation (Tulsa, Okla.). These linear alcoholic waxes are in general a mixture of linear alcohols having at least 20 carbon atoms and more particularly at least 24 carbon atoms.

The Vapour-Pressure Osmometry (VPO) technique can be used to characterize the number-average molecular weight of a mixture of alcohols. According to one particular form, the mixture of linear monoalcoholic waxes has a number-average molecular weight measured by VPO of approximately 200 to approximately 800, preferably of approximately 300 to approximately 600. A pure C22 monohydric linear alcohol has a molecular weight measured by VPO of 326.

In accordance with the present invention, use will preferably be made of a pure monoalcohol or a mixture of linear monoalcohols such as, for example: 1-eicosanol ($C_{20}$), 1-docosanol ($C_{22}$, behenyl alcohol), dotriacontanol ($C_{32}$), tetratriacontanol ($C_{34}$), pentatriacontanol ($C_{35}$), tetracontanol ($C_{40}$), tetraacontanol ($C_{44}$), dopentaacontanol ($C_{54}$), tetrahexaacontanol ($C_{64}$), dohexaacontanol ($C_{72}$).

Use will more particularly be made of 1-octadecanol, more commonly known as stearyl alcohol.

A final ingredient required for the preparation of the ETPEA resin is a polyol or polyhydric alcohol. The structure of the polyol is: $R_4(OH)_n$ where $R_4$ denotes an n-valent organic group. For example, $R_4$ may be a $C_2$-$C_{20}$ organic group with no hydroxyl substitution. As another example, $R_4$ may be a hydrocarbon-based group. n is in general equal to 2, 3, 4, 5 or 6.

Among the polyols that can be used according to the invention, mention may be made of ethylene glycol, propylene glycol, butylene glycol, glycerol, trimethylolpropane, pentaerythritol, neopentyl glycol, tris(hydroxylmethyl)methanol, dipentaerythritol and tripentaerythritol.

Neopentyl glycol will more particularly be used.

Reagents that are equivalents of the diacids and/or reagents that are equivalent to the diamines may also be used to prepare the ETPEA resin. For example, diesters may be used in place of some or all the diacids in the reaction forming the ETPEA resin. The term "diester" is intended to mean any product of esterification of a diacid with molecules comprising a hydroxyl function. Such diesters are preferably obtained from relatively volatile molecules containing hydroxyl functions in order for said molecules to be able to easily be removed from the reaction vessel after the reaction of the monoalcohol and/or of the diamine with the diester. A lower diester, in particular a product of esterification ordiesterification of a diacid as defined above with a $C_1$-$C_4$ monoalcohol (i.e.: methanol, ethanol, propanol and butanol), may be used in place of some or all the diacids in the reaction forming the ETPEA resin. Acid halides may also be used in place of some or all the diacids in the reaction forming the ETPEA resin. Likewise the monoalcohol can be esterified with a volatile diacid, for example: acetic acid, before being used in the reaction forming the ETPEA resin. Such equivalent reagents are not however preferential in so far as they introduce reactive groups into the reaction vessel.

Preferentially, the carboxylic acid equivalents must be substantially equal to the combined equivalents of hydroxyl introduced by the monoalcohol and the polyol and of amine introduced by the diamine. In other words, each of the acid numbers and amine numbers of the resin in accordance with the invention must preferably be less than 25, more preferentially less than 15 and more particularly less than 10, more particularly less than 5.

When a co-diacid is used to prepare the ETPEA resin, the co-diacid must not represent more than 50% of the carboxylic acid equivalents in the reaction mixture. In other words, the co-diacid represents from 0 to 50% equivalents, more preferentially from 0 to 25% and even more preferentially from 0 to 10% of the acid equivalents in the reaction mixture.

When a co-diamine is used to prepare the ETPEA resin, the co-diacid must not represent more than 50% of the carboxylic acid equivalents in the reaction mixture. In other words, the co-diamine represents from 0 to 50% equivalents, more preferentially from 0 to 25% and even more preferentially from 0 to 10% of the acid equivalents in the

reaction mixture.

The hydroxyl equivalents originating from the polyol are preferably less than or equal to 50% relative to all the hydroxyl equivalents and amine equivalents introduced by the polyol, monoalcohol and diamine reagents. According to one particular embodiment of the invention, the hydroxyl equivalents originating from the polyol may be less than or equal to 40%, or less than or equal to 30% or even less than or equal to 20% relative to all the hydroxyl equivalents and amine equivalents introduced by the polyol, monoalcohol and diamine reagents.

The amine equivalents preferably range from 0.3 to 0.75 relative to all the hydroxyl equivalents and amine equivalents introduced by the polyol, monoalcohol and diamine reagents. According to one particular embodiment of the invention, the hydroxyl equivalents originating from the polyol range from 0.05 to 0.45 relative to all the hydroxyl equivalents and amine equivalents introduced by the polyol, monoalcohol and diamine reagents. According to one particular embodiment of the invention, the hydroxyl equivalents originating from the monoalcohol range from 0.20 to 0.45 relative to all the hydroxyl equivalents and amine equivalents introduced by the polyol, monoalcohol and diamine reagents.

Use will more particularly be made of an ester-terminated poly(ester-amide) polymer having the INCI name Polyamide-8 which is a copolymer of hydrogenated dilinoleic acid, of ethylenediamine, of neopentyl glycol and of stearyl alcohol. This copolymer is in particular sold under the trade name Oleocrapht® LP-20-PA sold by the company Croda.

The ester-terminated poly(ester-amide) polymer(s) is (are) preferably present in the composition in a content ranging from 1% to 35%, better still from 2% to 30% by weight, preferably from 3% to 25% by weight, relative to the total weight of said composition.

## VOLATILE ALKANES

The compositions of the invention comprise at least one linear or branched volatile alkane.

The term "alkane" is intended to mean any compound comprising a linear or branched, saturated, hydrocarbon-based chain constituted exclusively of carbon atoms and hydrogen atoms.

The term "volatile alkane" that is suitable for use in the invention is intended to mean an alkane which is capable of evaporating on contact with the skin in less than one hour, at ambient temperature (25°C) and atmospheric pressure (760 mmHg, i.e. 101 325 Pa), which is liquid at ambient temperature, especially having an evaporation rate ranging from 0.01 to 15 mg/cm$^2$/minute, at ambient temperature (25°C) and atmospheric pressure (760 mmHg).

Among the volatile alkanes in accordance with the invention, mention may be made of:

Volatile linear alkanes;
Volatile branched alkanes;
and mixtures thereof.

a) Volatile linear alkanes

The volatile linear alkanes that are suitable for use in the invention are preferably chosen from volatile linear alkanes comprising from 7 to 14 carbon atoms.

As examples of alkanes that are suitable for the invention, mention may be made of the alkanes described in patent applications WO 2007/068371 and WO 2008/155059 from the company Cognis (mixtures of distinct alkanes differing by at least one carbon). These alkanes are obtained from fatty alcohols, which are themselves obtained from copra oil or palm oil.

As examples of linear alkanes that are suitable for use in the invention, mention may be made of n-heptane ($C_7$), n-octane ($C_8$), n-nonane ($C_9$), n-decane ($C_{10}$), n-undecane ($C_{11}$), n-dodecane ($C_{12}$), n-tridecane ($C_{13}$) and n-tetradecane ($C_{14}$), and mixtures thereof.

According to one preferred mode, mention may be made of mixtures of n-undecane ($C_{11}$) and of n-tridecane ($C_{13}$) obtained in Examples 1 and 2 of patent application WO 2008/155059 from the company Cognis.

Mention may also be made of n-dodecane ($C_{12}$) and n-tetradecane ($C_{14}$) sold by Sasol under the respective references Parafol 12 97 and Parafol 14 97, and also mixtures thereof.

b) Volatile branched alkanes

Among the branched volatile alkanes, mention may be made of branched $C_8$-$C_{16}$ alkanes, for instance $C_8$-$C_{16}$ isoalkanes of petroleum origin (also known as isoparaffins), for instance isododecane (also known as 2,2,4,4,6-pentamethylheptane), isodecane and isohexadecane, and mixtures thereof. Isododecane will preferably be used.

The volatile alkane(s) are preferably present in the composition in concentrations ranging from 35% to 95% by weight and more preferentially from 50% to 80% by weight relative to the total weight of the composition.

The compositions of the invention comprise at least one monoalcohol comprising from 2 to 8 carbon atoms, especially from 2 to 6 carbon atoms, and in particular from 2 to 4 carbon atoms.

The compositions of the invention may comprise one or more monoalcohol(s).

This monoalcohol can be represented for example by the formula RaOH, in which Ra represents a linear or branched alkyl group comprising from 2 to 8 carbon atoms.

As monoalcohol, mention may be made of ethanol, isopropanol, propanol or butanol.

According to one embodiment, the compositions of the invention comprise ethanol.

The amount of monoalcohol(s) varies preferentially from 2% to 20% by weight, and more preferentially from 3% to 15% by weight, relative to the total weight of said composition.

## PIGMENTS

The composition according to the invention comprises at least one pigment.

The term "pigments" means white or coloured, mineral or organic particles, which are insoluble in an aqueous medium, and which are intended to colour and/or opacify the resulting composition and/or deposit. These pigments may be white or coloured, and mineral and/or organic.

Preferably, the composition comprises at least 5% by weight of pigments, more preferentially from 5% to 40% by weight of pigments, in particular from 10% to 30% by weight and preferably from 10% to 20% by weight of pigments, relative to the total weight of said composition.

According to a particular embodiment, the pigments used according to the invention are chosen from mineral pigments.

The term "mineral pigment" is intended to mean any pigment that satisfies the definition in Ullmann's Encyclopaedia in the chapter on inorganic pigments. Among the mineral pigments that are useful in the present invention, mention may be made of zirconium oxide or cerium oxide, and also zinc oxide, iron oxide (black, yellow or red) or chromium oxide, manganese violet, ultramarine blue, chromium hydrate and ferric blue, titanium dioxide, and metal powders, for instance aluminium powder or copper powder. The following mineral pigments may also be used: $Ta_2O_5$, $Ti_3O_5$, $Ti_2O_3$, TiO, $ZrO_2$ as a mixture with $TiO_2$, $ZrO_2$, $Nb_2O_5$, $CeO_2$, ZnS.

The size of the pigment that is useful in the context of the present invention is generally greater than 100 nm and may range up to 10 μm, preferably from 200 nm to 5 μm and more preferentially from 300 nm to 1 μm.

According to a particular form of the invention, the pigments have a size characterized by a D[50] greater than 100 nm and possibly ranging up to 10 μm, preferably from 200 nm to 5 μm and more preferentially from 300 nm to 1 μm.

The sizes are measured by static light scattering using a commercial MasterSizer 3000 particle size analyser from Malvern, which makes it possible to determine the particle size distribution of all of the particles over a wide range which may extend from 0.01 μm to 1000 μm. The data are processed on the basis of the standard Mie scattering theory. This theory is the most suitable for size distributions ranging from submicron to multimicron; it allows an "effective" particle diameter to be determined.

This theory is especially described in the publication by Van de Hulst, H.C., Light Scattering by Small Particles, Chapters 9 and 10, Wiley, New York, 1957.

D[50] represents the maximum size that 50% by volume of the particles have.

In the context of the present invention, the mineral pigments are more particularly chosen from iron oxides and/or titanium dioxides, and more preferentially iron oxide and/or titanium dioxide pigments coated with at least one lipophilic or hydrophobic compound.

Examples that may be mentioned more particularly include titanium dioxides and iron oxide coated with aluminium stearoyl glutamate, sold, for example, under the reference NAI by the company Miyoshi Kasei.

As mineral pigments that may be used in the invention, mention may also be made of nacres.

The term "nacres" should be understood as meaning coloured particles of any form, which may or may not be iridescent, especially produced by certain molluscs in their shell, or alternatively synthesized, and which have a colour effect via optical interference.

The nacres may be chosen from nacreous pigments such as titanium mica coated with an iron oxide, titanium mica coated with bismuth oxychloride, titanium mica coated with chromium oxide, titanium mica coated with an organic dye and also nacreous pigments based on bismuth oxychloride. They may also be mica particles at the surface of which are superposed at least two successive layers of metal oxides and/or of organic colorants.

Examples of nacres that may also be mentioned include natural mica coated with titanium oxide, with iron oxide, with natural pigment or with bismuth oxychloride. Among the nacres available on the market, mention may be made of the nacres Timica, Flamenco and Duochrome (based on mica) sold by the company Engelhard, the Timiron nacres sold by the company Merck, the Prestige mica-based nacres sold by the company Eckart, and the Sunshine synthetic mica-based nacres sold by the company Sun Chemical.

The nacres may more particularly have a yellow, pink, red, bronze, orange, brown, gold and/or coppery colour

or tint.

As illustrations of nacres that may be used in the context of the present invention, mention may be made especially of the gold-coloured nacres sold especially by the company Engelhard under the name Brilliant gold 212G (Timica), Gold 222C (Cloisonne), Sparkle gold (Timica), Gold 4504 (Chromalite) and Monarch gold 233X (Cloisonne); the bronze nacres sold especially by the company Merck under the name Bronze fine (17384) (Colorona) and Bronze (17353) (Colorona) and by the company Engelhard under the name Super bronze (Cloisonne); the orange nacres sold especially by the company Engelhard under the name Orange 363C (Cloisonne) and Orange MCR 101 (Cosmica) and by the company Merck under the name Passion orange (Colorona) and Matte orange (17449) (Microna); the brown nacres sold especially by the company Engelhard under the name Nu-antique copper 340XB (Cloisonne) and Brown CL4509 (Chromalite); the nacres with a copper tint sold especially by the company Engelhard under the name Copper 340A (Timica); the nacres with a red tint sold especially by the company Merck under the name Sienna fine (17386) (Colorona); the nacres with a yellow tint sold especially by the company Engelhard under the name Yellow (4502) (Chromalite); the red nacres with a gold tint sold especially by the company Engelhard under the name Sunstone G012 (Gemtone); the pink nacres sold especially by the company Engelhard under the name Tan opale G005 (Gemtone); the black nacres with a gold tint sold especially by the company Engelhard under the name Nu antique bronze 240 AB (Timica), the blue nacres sold especially by the company Merck under the name Matte blue (17433) (Microna), the white nacres with a silvery tint sold especially by the company Merck under the name Xirona Silver, and the golden-green pink-orange nacres sold especially by the company Merck under the name Indian summer (Xirona), and mixtures thereof.

Among the pigments that may be used according to the invention, mention may also be made of those having an optical effect different from a simple conventional colouring effect, i.e. a unified and stabilized effect such as produced by conventional colorants, for instance monochromatic pigments. For the purposes of the invention, the term "stabilized" means lacking the effect of variability of the colour with the angle of observation or in response to a temperature change.

For example, this material may be chosen from particles with a metallic tint, goniochromatic colouring agents, diffractive pigments, thermochromic agents, optical brighteners, and also fibres, in particular interference fibres. Needless to say, these various materials may be combined in order simultaneously to afford two effects, or even a novel effect in accordance with the invention.

The particles with a metallic tint that may be used in the invention are in particular chosen from:

- particles of at least one metal and/or of at least one metal derivative,
- particles comprising a monomaterial or multimaterial organic or mineral substrate, at least partially coated with at least one layer with a metallic tint comprising at least one metal and/or at least one metal derivative, and mixtures of said particles.

Among the metals that may be present in said particles, mention may for example be made of Ag, Au, Cu, Al, Ni, Sn, Mg, Cr, Mo, Ti, Zr, Pt, Va, Rb, W, Zn, Ge, Te and Se, and mixtures or alloys thereof. Ag, Au, Cu, Al, Zn, Ni, Mo, Cr and mixtures or alloys thereof (for example, bronzes and brasses) are preferred metals.

The term "metal derivatives" denotes compounds derived from metals, especially oxides, fluorides, chlorides and sulfides.

By way of illustration of these particles, mention may be made of particles of aluminium, such as those sold under the names Starbrite 1200 EAC® by the company Silberline and Metalure® by the company Eckart.

Mention may also be made of metal powders of copper or of alloy mixtures such as the references 2844 sold by the company Radium Bronze, metallic pigments, for instance aluminium or bronze, such as those sold under the names Rotosafe 700 from the company Eckart, silica-coated aluminium particles sold under the name Visionaire Bright Silver from the company Eckart, and metal alloy particles, for instance the silica-coated bronze (alloy of copper and zinc) powders sold under the name Visionaire Bright Natural Gold from the company Eckart.

They may also be particles comprising a glass substrate, for instance those sold by the company Nippon Sheet Glass under the name Microglass Metashine®.

The goniochromatic colouring agent may be chosen, for example, from multilayer interference structures and liquid-crystal colouring agents.

Examples of symmetrical multilayer interference structures that may be used in the compositions prepared in accordance with the invention are, for example, the following structures: $Al/SiO_2/Al/SiO_2/Al$, pigments having this structure being sold by the company DuPont de Nemours; $Cr/MgF_2/Al/MgF_2/Cr$, pigments having this structure being sold under the name Chromaflair by the company Flex; $MoS_2/SiO_2/Al/SiO_2/MoS_2$; $Fe_2O_3/SiO_2/Al/SiO_2/Fe_2O_3$, and $Fe_2O_3/SiO_2/Fe_2O_3/SiO_2/Fe_2O_3$, pigments having these structures being sold under the name Sicopearl by the company BASF; $MoS_2/SiO_2$/mica-oxide/$SiO_2/MoS_2$; $Fe_2O_3/SiO_2$/mica-oxide/$SiO_2/Fe_2O_3$; $TiO_2/SiO_2/TiO_2$ and $TiO_2/Al_2O_3/TiO_2$; $SnO/TiO_2/SiO_2/TiO_2/SnO$; $Fe_2O_3/SiO_2/Fe_2O_3$; SnO/mica/$TiO_2/SiO_2/TiO_2$/mica/SnO, pigments having these structures being sold under the name Xirona by the company Merck (Darmstadt). By way of example, these pigments may be the pigments of silica/titanium oxide/tin oxide structure sold under the name Xirona Magic by the company Merck, the

pigments of silica/brown iron oxide structure sold under the name Xirona Indian Summer by the company Merck and the pigments of silica/titanium oxide/mica/tin oxide structure sold under the name Xirona Caribbean Blue by the company Merck. Mention may also be made of the Infinite Colors pigments from the company Shiseido. Depending on the thickness and the nature of the various coats, different effects are obtained. Thus, with the structure $Fe_2O_3/SiO_2/Al/SiO_2/Fe_2O_3$, the colour changes from green-golden to red-grey for $SiO_2$ layers of 320 to 350 nm; from red to golden for $SiO_2$ layers of 380 to 400 nm; from purple to green for $SiO_2$ layers of 410 to 420 nm; and from copper to red for $SiO_2$ layers of 430 to 440 nm.

As examples of pigments with a polymeric multilayer structure, mention may be made of those sold by the company 3M under the name Color Glitter.

Examples of liquid-crystal goniochromatic particles that may be used include those sold by the company Chenix and also the product sold under the name Helicone® HC by the company Wacker.

Hydrophobic coated pigments

Preferably, the compositions according to the invention comprise at least one pigment coated with at least one lipophilic or hydrophobic compound and especially as detailed below.

This type of pigment is particularly advantageous in so far as it may be considered in a large amount together with a large amount of water. What is more, in so far as they are treated with a hydrophobic compound, they show a predominant affinity for the oily gelled phase, which can then convey them.

Needless to say, the compositions according to the invention may in parallel contain uncoated pigments.

The coating may also comprise at least one additional non-lipophilic compound. For the purposes of the invention, the "coating" of a pigment according to the invention generally denotes the total or partial surface treatment of the pigment with a surface agent absorbed, adsorbed or grafted onto said pigment.

The surface-treated pigments may be prepared according to surface treatment techniques of chemical, electronic, mechanochemical or mechanical nature that are well known to those skilled in the art. Commercial products may also be used.

The surface agent may be absorbed, adsorbed or grafted onto the pigments by evaporation of solvent, chemical reaction and creation of a covalent bond.

According to one variant, the surface treatment is constituted of a coating of the pigments.

The coating may represent from 0.1 % to 20% by weight and in particular from 0.5% to 5% by weight of the total weight of the coated pigment.

The coating may be realized, for example, by adsorption of a liquid surface agent onto the surface of the solid particles by simple mixing with stirring of the particles and of said surface agent, optionally with heating, prior to the incorporation of the particles into the other ingredients of the makeup or care composition.

The coating may be realized, for example, by chemical reaction of a surface agent with the surface of the solid pigment particles and creation of a covalent bond between the surface agent and the particles. This method is especially described in the patent US 4,578,266.

The chemical surface treatment may consist in diluting the surface agent in a volatile solvent, dispersing the pigments in this mixture and then slowly evaporating off the volatile solvent, so that the surface agent is deposited at the surface of the pigments.

Lipophilic or hydrophobic treatment agent

When the pigment comprises a lipophilic or hydrophobic coating, it is preferably present in the fatty phase of the composition according to the invention.

According to a particular embodiment of the invention, the pigments may be coated according to the invention with at least one compound chosen from silicone surface agents; fluoro surface agents; fluorosilicone surface agents; metal soaps; N-acylamino acids or salts thereof; lecithin and derivatives thereof; isopropyl triisostearyl titanate; isostearyl sebacate; natural plant or animal waxes; polar synthetic waxes; fatty esters; phospholipids; and mixtures thereof.

Silicone surface agent

According to a particular embodiment, the pigments may be totally or partially surface-treated with a compound of silicone nature.

The silicone surface agents may be chosen from organopolysiloxanes, silane derivatives, silicone-acrylate copolymers, silicone resins, and mixtures thereof.

The term "organopolysiloxane compound" is intended to mean a compound having a structure comprising an alternance of silicone atoms and oxygen atoms and comprising organic radicals linked to the silicon atoms.

i) Non-elastomeric organopolysiloxane

Non-elastomeric organopolysiloxanes that may especially be mentioned include polydimethylsiloxanes, polymethylhydrogenosiloxanes and polyalkoxydimethylsiloxanes.

The alkoxy group may be represented by the radical R-O- such that R represents methyl, ethyl, propyl, butyl or octyl, 2-phenylethyl, 2-phenylpropyl or 3,3,3-trifluoropropyl radicals, aryl radicals such as phenyl, tolyl or xylyl, or substituted aryl radicals such as phenylethyl.

One method for surface-treating pigments with a polymethylhydrogenosiloxane consists in dispersing the pigments in an organic solvent and then in adding the silicone compound. On heating the mixture, covalent bonds are created between the silicone compound and the surface of the pigment.

According to a preferred embodiment, the silicone surface agent may be a non-elastomeric organopolysiloxane, especially chosen from polydimethylsiloxanes.

ii) Alkylsilanes and alkoxysilanes

Silanes bearing alkoxy functionality are especially described by Witucki in "A silane primer, Chemistry and applications of alkoxy silanes, Journal of Coatings Technology, 65, 822, pages 57-60, 1993".

Alkoxysilanes such as the alkyltriethoxysilanes and the alkyltrimethoxysilanes sold under the references Silquest A-137 (OSI Specialities) and Prosil 9202 (PCR) may be used for coating the pigments.

The use of alkylpolysiloxanes bearing a reactive end group such as alkoxy, hydroxyl, halogen, amino or imino is described in application JP H07-196946. They are also suitable for treating the pigments.

iii) Silicone-acrylate polymers

Grafted silicone-acrylic polymers having a silicone backbone as described in patents US 5 725 882, US 5 209 924, US 4 972 037, US 4 981 903, US 4 981 902 and US 5 468 477 and in patents US 5 219 560 and EP 0 388 582 may be used.

Other silicone-acrylate polymers may be silicone polymers comprising in their structure the unit of formula (I) below:

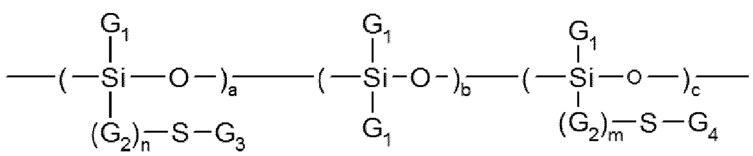

(I)

in which the radicals $G_1$, which may be identical or different, represent hydrogen or a $C_1$-$C_{10}$ alkyl radical or alternatively a phenyl radical; the radicals $G_2$, which may be identical or different, represent a $C_1$-$C_{10}$ alkylene group; $G_3$ represents a polymeric residue resulting from the (homo)polymerization of at least one ethylenically unsaturated anionic monomer; $G_4$ represents a polymeric residue resulting from the (homo)polymerization of at least one ethylenically unsaturated hydrophobic monomer; m and n are equal to 0 or 1; a is an integer ranging from 0 to 50; b is an integer that may be between 10 and 350, c is an integer ranging from 0 to 50; with the proviso that one of the parameters a and c is other than 0.

Preferably, the unit of formula (I) above has at least one, and even more preferentially all, of the following characteristics:

- the radicals G1 denote an alkyl radical, preferably a methyl radical;
- n is non-zero, and the radicals G2 represent a divalent C1-C3 radical, preferably a propylene radical;
- G3 represents a polymeric radical resulting from the (homo)polymerization of at least one monomer of the ethylenically unsaturated carboxylic acid type, preferably acrylic acid and/or methacrylic acid;
- G4 represents a polymeric radical resulting from the (homo)polymerization of at least one monomer of the (C1-C10)alkyl (meth)acrylate type, preferably such as isobutyl or methyl (meth)acrylate.

Examples of silicone polymers corresponding to formula (I) are especially polydimethylsiloxanes (PDMS) onto which are grafted, via a connecting chain unit of thiopropylene type, mixed polymer units of the poly(meth)acrylic acid type and of the polymethyl (meth)acrylate type.

Other examples of silicone polymers corresponding to formula (I) are especially polydimethylsiloxanes (PDMS) onto which are grafted, via a connecting chain unit of thiopropylene type, polymer units of the polyisobutyl (meth)acrylate type.

iv) Silicone resins

The silicone surface agent may be chosen from silicone resins.

The term "resin" means a three-dimensional structure.

The silicone resins may be soluble or swellable in silicone oils. These resins are crosslinked polyorganosiloxane polymers.

The nomenclature of silicone resins is known under the name "MDTQ", the resin being described as a function of the various siloxane monomer units that it comprises, each of the letters "MDTQ" characterizing a type of unit.

The letter M represents the monofunctional unit of formula $(CH_3)_3SiO_{1/2}$, the silicon atom being bonded to only one oxygen atom in the polymer comprising this unit.

The letter D means a difunctional unit $(CH_3)_2SiO_{2/2}$ in which the silicon atom is bonded to two oxygen atoms.

The letter T represents a trifunctional unit of formula $(CH_3)SiO_{3/2}$.

In the units M, D and T defined previously, at least one of the methyl groups may be substituted with a group R other than the methyl group, such as a hydrocarbon-based radical (especially alkyl) containing from 2 to 10 carbon atoms or a phenyl group, or alternatively a hydroxyl group.

Finally, the letter Q means a tetrafunctional unit $SiO_{4/2}$ in which the silicon atom is bonded to four hydrogen atoms, which are themselves bonded to the rest of the polymer.

Various resins with different properties may be obtained from these different units, the properties of these polymers varying as a function of the type of monomers (or units), of the type and number of substituted radicals, of the length of the polymer chain, of the degree of branching and of the size of the side chains.

Examples of these silicone resins that may be mentioned include:

- siloxysilicates, which may be trimethyl siloxysilicates of formula $[(CH_3)_3XSiXO]_xX(SiO_{4/2})_y$ (MQ units) in which x and y are integers ranging from 50 to 80;
- polysilsesquioxanes of formula $(CH_3SiO_{3/2})_x$ (T units) in which x is greater than 100 and at least one of the methyl radicals of which may be substituted with a group R as defined above;
- polymethylsilsesquioxanes, which are polysilsesquioxanes in which none of the methyl radicals is substituted with another group.

Such polymethylsilsesquioxanes are described in US 5 246 694.

As examples of commercially available polymethylsilsesquioxane resins, mention may be made of those sold:

- by the company Wacker under the reference Resin MK, such as Belsil PMS MK: polymer comprising $CH_3SiO_{3/2}$ repeating units (T units), which may also comprise up to 1% by weight of $(CH_3)_2SiO_{2/2}$ units (D units) and having an average molecular weight of about 10 000;
- by the company Shin-Etsu under the references KR-220L, which are composed of units T of formula $CH_3SiO_{3/2}$ and contain Si-OH (silanol) end groups, under the reference KR-242A, which comprise 98% of units T and 2% of dimethyl units D and contain Si-OH end groups, or else under the reference KR-251, which comprise 88% of units T and 12% of dimethyl units D and contain Si-OH end groups.

Siloxysilicate resins that may be mentioned include trimethylsiloxysilicate (TMS) resins, optionally in the form of powders. Such resins are sold under the references SR1000, E 1 170-002 or SS 4230 by the company General Electric or under the references TMS 803, Wacker 803 and 804 by the company Wacker Silicone Corporation.

Mention may also be made of trimethylsiloxysilicate resins sold in a solvent such as cyclomethicone, sold under the name KF-7312J by the company Shin-Etsu or DC 749 and DC 593 by the company Dow Corning.

As examples of commercial references of pigments treated with a silicone compound, mention may be made of:

- red iron oxide/dimethicone sold under the reference SA-C 338075-10 by the company Miyoshi Kasei, and
- a pigment obtained by treating DC Red 7 with a silicone compound, sold by the company Coletica under the reference Gransil GCM (which is a mixture of D5 and polysilicone 11).

Fluoro surface agent

The pigments may be totally or partially surface-treated with a compound of fluoro nature.

The fluoro surface agents may be chosen from perfluoroalkyl phosphates, perfluoropolyethers, polytetrafluoropolyethylenes (PTFE), perfluoroalkanes, perfluoroalkyl silazanes, polyhexafluoropropylene oxides, and polyorganosiloxanes comprising perfluoroalkyl perfluoropolyether groups.

The term "perfluoroalkyl radical" is intended to mean an alkyl radical in which all the hydrogen atoms have been

replaced with fluorine atoms.

Perfluoropolyethers are especially described in patent application EP 0 486 135, and sold under the trade name Fomblin by the company Montefluos.

Perfluoroalkyl phosphates are in particular described in application JP H05-86984. The perfluoroalkyl diethanolamine phosphates sold by Asahi Glass under the reference AsahiGuard AG530 may be used.

Among the linear perfluoroalkanes that may be mentioned are perfluorocycloalkanes, perfluoro(alkylcycloalkanes), perfluoropolycycloalkanes, aromatic perfluoro hydrocarbons (perfluoroarenes) and hydrocarbon-based perfluoro organic compounds comprising at least one heteroatom.

Among the perfluoroalkanes, mention may be made of the linear alkane series such as perfluorooctane, perfluorononane or perfluorodecane.

Among the perfluorocycloalkanes and perfluoro(alkylcycloalkanes), mention may be made of perfluorodecalin sold under the name Flutec PP5 GMP by the company Rhodia, perfluoro(methyldecalin) and perfluoro(C3-C5 alkylcyclohexanes) such as perfluoro(butylcyclohexane).

Among the perfluoropolycycloalkanes, mention may be made of bicyclo[3.3.1]nonane derivatives such as perfluorotrimethylbicyclo[3.3.1]nonane, adamantane derivatives such as perfluorodimethyladamantane, and hydrogenated perfluorophenanthrene derivatives such as tetracosafluorotetradecahydrophenanthrene.

Among the perfluoroarenes, mention may be made of perfluoronaphthalene derivatives, for instance perfluoronaphthalene and perfluoromethyl-1-naphthalene.

As examples of commercial references of pigments treated with a fluoro compound, mention may be made of:

- yellow iron oxide/perfluoroalkyl phosphate sold under the reference PF 5 Yellow 601 by the company Daito Kasei;
- red iron oxide/perfluoroalkyl phosphate sold under the reference PF 5 Red R 516L by the company Daito Kasei;
- black iron oxide/perfluoroalkyl phosphate sold under the reference PF 5 Black BL 100 by the company Daito Kasei;
- titanium dioxide/perfluoroalkyl phosphate sold under the reference PF 5 TiO2 CR 50 by the company Daito Kasei;
- yellow iron oxide/perfluoropolymethyl isopropyl ether sold under the reference Iron oxide yellow BF-25-3 by the company Toshiki;
- DC Red 7/perfluoropolymethyl isopropyl ether sold under the reference D&C Red 7 FHC by the company Cardre Inc.; and
- DC Red 6/PTFE sold under the reference T 9506 by the company Warner-Jenkinson.

Fluorosilicone surface agent

The pigments may be totally or partially surface-treated with a compound of fluorosilicone nature.

The fluorosilicone compound may be chosen from perfluoroalkyl dimethicones, perfluoroalkyl silanes and perfluoroalkyl trialkoxysilanes.

Perfluoroalkyl silanes that may be mentioned include the products LP-IT and LP-4T sold by Shin-Etsu Silicone.

The perfluoroalkyl dimethicones may be represented by the following formula:

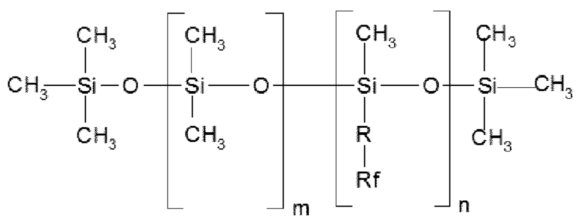

in which:

- R represents a linear or branched divalent alkyl group containing from 1 to 6 carbon atoms, preferably a divalent methyl, ethyl, propyl or butyl group;
- Rf represents a perfluoroalkyl radical containing 1 to 9 carbon atoms and preferably 1 to 4 carbon atoms;
- m is chosen between 0 and 150 and preferably between 20 and 100; and
- n is chosen between 1 and 300 and preferably between 1 and 100.

As examples of commercial references of pigments treated with a fluorosilicone compound, mention may be made of titanium dioxide/fluorosilicone sold under the reference Fluorosil Titanium dioxide 100TA by the company Advanced Dermaceuticals International Inc.

Other lipophilic surface agents

The hydrophobic treating agent may also be chosen from:

i) metal soaps such as aluminium dimyristate and the aluminium salt of hydrogenated tallow glutamate;

Metal soaps that may especially be mentioned include metal soaps of fatty acids containing from 12 to 22 carbon atoms and in particular those containing from 12 to 18 carbon atoms.

The metal of the metal soap may especially be zinc or magnesium.

Metal soaps that may be used include zinc laurate, magnesium stearate, magnesium myristate and zinc stearate, and mixtures thereof;

ii) fatty acids such as lauric acid, myristic acid, stearic acid and palmitic acid;
iii) N-acylamino acids or salts thereof, which may comprise an acyl group containing from 8 to 22 carbon atoms, for instance a 2-ethylhexanoyl, caproyl, lauroyl, myristoyl, palmitoyl, stearoyl or cocoyl group;

The amino acid may be, for example, lysine, glutamic acid or alanine.

The salts of these compounds may be the aluminium, magnesium, calcium, zirconium, zinc, sodium or potassium salts.

Thus, according to a particularly preferred embodiment, an N-acylamino acid derivative may especially be a glutamic acid derivative and/or a salt thereof, and more particularly a stearoyl glutamate, for instance aluminium stearoyl glutamate. iv) lecithin and derivatives thereof;
v) isopropyl triisostearyl titanate.

As examples of isopropyl titanium triisostearate (ITT)-treated pigments, mention may be made of those sold under the commercial references BWBO-I2 (Iron oxide CI77499 and isopropyl titanium triisostearate), BWYO-I2 (Iron oxide CI77492 and isopropyl titanium triisostearate) and BWRO-I2 (Iron oxide CI77491 and isopropyl titanium triisostearate) by the company Kobo;

vi) isostearyl sebacate;
vii) natural plant or animal waxes or polar synthetic waxes;
viii) fatty esters, in particular jojoba esters;
ix) phospholipids; and
x) mixtures thereof.

The waxes mentioned in the compounds mentioned previously may be those generally used in cosmetics, as defined hereinbelow.

They may especially be hydrocarbon-based, silicone and/or fluoro waxes, optionally comprising ester or hydroxyl functions. They may also be of natural or synthetic origin.

The term "polar wax" is intended to mean a wax containing chemical compounds comprising at least one polar group. Polar groups are well known to those skilled in the art; they may be, for example, alcohol, ester or carboxylic acid groups. Polyethylene waxes, paraffin waxes, microcrystalline waxes, ozokerite and Fischer-Tropsch waxes are not included among polar waxes.

In particular, the polar waxes have a mean Hansen solubility parameter δa at 25°C such that $\delta a > 0\ (J/cm^3)^{1/2}$ and better still $\delta a > 1\ (J/cm^3)^{1/2}$:

$$\delta_a = \sqrt{\delta_p^2 + \delta_h^2}$$

in which δp and δh are, respectively, the polar contributions and contributions of interaction types specific to the Hansen solubility parameters.

The definition of solvents in the three-dimensional solubility space according to Hansen is described in the article by C.M. Hansen: "The three-dimensional solubility parameters", J. Paint Technol. 39, 105 (1967):

- δh characterizes the specific interaction forces (such as hydrogen bonding, acid/base, donor/acceptor, etc.);
- δp characterizes the Debye interaction forces between permanent dipoles and also the Keesom interaction forces between induced dipoles and permanent dipoles.

The parameters δp and δh are expressed in $(J/cm^3)^{1/2}$.

A polar wax is especially formed from molecules comprising, besides carbon and hydrogen atoms in their chemical structure, heteroatoms (such as O, N and P).

Non-limiting illustrations of these polar waxes that may especially be mentioned include natural polar waxes, such as beeswax, lanolin wax, orange wax, lemon wax and Chinese insect waxes, rice bran wax, carnauba wax, candelilla wax, ouricury wax, cork fibre wax, sugarcane wax, Japan wax, sumac wax and montan wax.

According to a particular embodiment, the pigments may be coated with at least one compound chosen from silicone surface agents; fluoro surface agents; N-acylamino acids or salts thereof; isopropyl triisostearyl titanate; natural plant or animal waxes; fatty esters; and mixtures thereof.

According to a particularly preferred embodiment, the pigments may be coated with an N-acylamino acid and/or a salt thereof, in particular with a glutamic acid derivative and/or a salt thereof, or with a fatty ester, in particular with a jojoba ester.

According to a more particularly preferred embodiment, the pigments may be coated with an N-acylamino acid and/or a salt thereof, in particular with a glutamic acid derivative and/or a salt thereof, especially a stearoyl glutamate, for instance aluminium stearoyl glutamate.

Examples of coated pigments according to the invention that may be mentioned more particularly include titanium dioxides and iron oxide coated with aluminium stearoyl glutamate, sold, for example, under the reference NAI by Miyoshi Kasei.

Pigments not coated with a hydrophobic compound

As stated previously, a composition may also contain pigments not coated with a lipophilic or hydrophobic compound.

These other pigments may be coated with a hydrophilic compound or uncoated.

These pigments may be mineral pigments especially as defined previously.

These pigments may also be organic pigments.

The term "organic pigment" is intended to mean any pigment that satisfies the definition in Ullmann's Encyclopaedia in the chapter on organic pigments. The organic pigment may in particular be chosen from nitroso, nitro, azo, xanthene, quinoline, anthraquinone, phthalocyanine, metal complex type, isoindolinone, isoindoline, quinacridone, perinone, perylene, diketopyrrolopyrrole, thioindigo, dioxazine, triphenylmethane and quinophthalone compounds.

The organic pigment(s) may be chosen, for example, from carmine, carbon black, aniline black, melanin, azo yellow, quinacridone, phthalocyanine blue, sorghum red, the blue pigments codified in the Color Index under the references CI 42090, 69800, 69825, 73000, 74100 and 74160, the yellow pigments codified in the Color Index under the references CI 11680, 11710, 15985, 19140, 20040, 21100, 21108, 47000 and 47005, the green pigments codified in the Color Index under the references CI 61565, 61570 and 74260, the orange pigments codified in the Color Index under the references CI 11725, 15510, 45370 and 71105, the red pigments codified in the Color Index under the references CI 12085, 12120, 12370, 12420, 12490, 14700, 15525, 15580, 15620, 15630, 15800, 15850, 15865, 15880, 17200, 26100, 45380, 45410, 58000, 73360, 73915 and 75470, and the pigments obtained by oxidative polymerization of indolic or phenolic derivatives as described in patent FR 2 679 771.

These pigments may also be in the form of composite pigments as described in patent EP 1 184 426. These composite pigments may especially be composed of particles comprising a mineral core at least partially covered with an organic pigment and at least one binder for fixing the organic pigments to the core.

The pigment may also be a lake. The term "lake" is intended to mean insolubilized dyes adsorbed onto insoluble particles, the assembly thus obtained remaining insoluble during use.

The inorganic substrates onto which the dyes are adsorbed are, for example, alumina, silica, calcium sodium borosilicate, calcium aluminium borosilicate and aluminium.

Among the organic dyes, mention may be made of cochineal carmine. Mention may also be made of the products known under the following names: D&C Red 21 (CI 45 380), D&C Orange 5 (CI 45 370), D&C Red 27 (CI 45 410), D&C Orange 10 (CI 45 425), D&C Red 3 (CI 45 430), D&C Red 4 (CI 15 510), D&C Red 33 (CI 17 200), D&C Yellow 5 (CI 19 140), D&C Yellow 6 (CI 15 985), D&C Green (CI 61 570), D&C Yellow 10 (CI 77 002), D&C Green 3 (CI 42 053), D&C Blue 1 (CI 42 090).

An example of a lake that may be mentioned is the product known under the name D&C Red 7 (CI 15 850:1).

Nature of the hydrophilic coating

As stated previously, these other pigments may be coated with a hydrophilic compound.

Said hydrophilic compound for surface-treating a pigment in order to optimize its dispersion in the gelled aqueous phase is more particularly chosen from biological polymers, carbohydrates, polysaccharides, polyacrylates and polyethylene glycol derivatives.

As examples of biological polymers, mention may be made of polymers based on monomers of carbohydrate type.

More particularly, mention may be made of biosaccharide gum; chitosans and derivatives thereof, such as butoxy chitosan, carboxymethyl chitosan, carboxybutyl chitosan, chitosan gluconate, chitosan adipate, chitosan glycolate, chitosan lactate, chitins and derivatives thereof, such as carboxymethyl chitin, chitin glycolate; cellulose and derivatives thereof such as cellulose acetate; microcrystalline cellulose; distarch phosphate; sodium hyaluronate; soluble proteoglycans; galacto-arabinans; glycosaminoglycans; glycogen; sclerotium gum; dextran; starch and derivatives thereof; and mixtures thereof.

Examples of carbohydrates that may especially be mentioned include polyhydroxyaldehydes and polyhydroxy ketones of general formula: $C_x(H_2O)_y$ in which x and y may range from 1 to 1 000 000.

The carbohydrates may be monosaccharides, disaccharides or polysaccharides.

Examples of carbohydrates that may especially be mentioned include amylodextrins, beta-glucans, cyclodextrins, modified corn starch, glycogen, hyaluronic acid, hydroxypropylcyclodextrin, lactose, maltitol, guanosine, glyceryl starch, *Triticum vulgare* starch, trehalose, sucrose and derivatives thereof, raffinose and sodium chondroitin sulfate.

$C_1$-$C_{20}$ alkylene glycols or $C_1$-$C_{20}$ alkylene glycol ethers, alone or used in combination with tri($C_1$-$C_{20}$)alkylsilanes, may also be used as surface treatment agents.

Examples that may be mentioned include pigments surface-treated with PEG alkyl ether alkoxysilane, for instance pigments treated with PEG-8-methyl ether triethoxysilane sold by the company Kobo under the name SW pigments.

Silicones such as dimethicones bearing hydrophilic groups, also known under the name dimethicone copolyols or alkyl dimethicone copolyols, may also be suitable for use in the invention as surface treatment agents. In particular, such dimethicones may comprise, as repeating units, $C_1$-$C_{20}$ alkylene oxides, such as ethylene or propylene oxides.

An example that may be mentioned is the pigment treated with PEG-12-dimethicone, sold by the company Sensient Corporation under the name LCW AQ Pigment.

The amount of pigments coated with at least one hydrophilic compound and/or of uncoated pigments is especially conditioned by the intended use of the cosmetic composition under consideration, and the adjustment of this amount obviously falls within the competence of the composition formulator.

According to one particularly preferred embodiment, the composition of the invention comprises at least one pigment coated with at least one lipophilic or hydrophobic compound, in particular iron oxide and/or titanium dioxide pigments coated with at least one lipophilic or hydrophobic compound.

According to a particularly preferred embodiment, the pigments may be coated with an N-acylamino acid and/or a salt thereof, in particular with a glutamic acid derivative and/or a salt thereof, or with a fatty ester, in particular with a jojoba ester.

According to a more particularly preferred embodiment, the pigments may be coated with an N-acylamino acid and/or a salt thereof, in particular with a glutamic acid derivative and/or a salt thereof, especially a stearoyl glutamate, for instance aluminium stearoyl glutamate.

Examples of coated pigments according to the invention that may be mentioned more particularly include titanium dioxides and/or iron oxides coated with aluminium stearoyl glutamate, sold, for example, under the reference NAI by Miyoshi Kasei.

According to one particularly preferred embodiment, the anhydrous fluid composition of the invention comprises

a) at least one polymer having the INCI name Polyamide-8; and

b) at least one $C_8$-$C_{16}$ branched volatile alkane, in particular chosen from isododecane, isodecane, isohexadecane and mixtures thereof and more particularly isododecane; and

c) at least ethanol;

d) a particulate phase comprising at least one pigment chosen from iron oxides and/or titanium dioxides, and more preferentially iron oxide and/or titanium dioxide pigments coated with at least one lipophilic or hydrophobic compound.

## COSMETIC COMPOSITIONS

The present invention also relates to a cosmetic composition comprising, in a physiologically acceptable medium, a composition as defined above.

The term "physiologically acceptable medium" is intended to denote a medium that is particularly suitable for the application of a composition of the invention to the skin.

The physiologically acceptable medium is generally adapted to the nature of the support onto which the composition has to be applied, and also to the appearance under which the composition has to be packaged.

For the purposes of the present invention, the term "anhydrous" refers to a composition comprising a content

of less than or equal to 2% by weight, preferably less than or equal to 1 % and more preferentially less than 0.5% by weight of water relative to the total weight of said composition, or is even free of water. Where appropriate, such small amounts of water may especially be introduced by ingredients of the composition that may contain residual amounts thereof.

## ADDITIVES

The compositions according to the invention may in addition comprise additives commonly used in care and/or makeup products, such as:

- active agents such as vitamins, for example vitamins A, E, C, $B_3$;
- moisturizing agents;
- sunscreens;
- fillers;
- additional colorants;
- fragrances;
- preservatives;
- and mixtures thereof.

It is a matter of routine operation for those skilled in the art to adjust the nature and the amount of the additives present in the compositions in accordance with the invention such that the desired cosmetic properties thereof are not thereby affected.

### Fillers

The compositions in accordance with the invention may also comprise at least one filler, of organic or mineral nature, which can especially give them complementary properties of mattness, coverage, persistence and/or improved stability.
The term "filler" should be understood as meaning colourless or white solid particles of any form, which are in an insoluble form dispersed in the medium of the composition. These particles, of mineral or organic nature, give body or rigidity to the composition and/or softness and uniformity to the makeup.
The fillers used in the compositions according to the present invention can be of lamellar, globular, spherical or fibrous forms or of any other form intermediate between these defined forms.
The fillers according to the invention may or may not be surface-coated, and in particular they may be surface-treated with silicones, amino acids, fluorinated derivatives or any other substance which promotes the dispersion and the compatibility of the filler in the composition.
Examples of mineral fillers that may be mentioned include talc, mica, silica, hollow silica microspheres, kaolin, calcium carbonate, magnesium carbonate, hydroxyapatite, boron nitride, glass or ceramic microcapsules, or composites of silica and of titanium dioxide, for instance the TSG series sold by Nippon Sheet Glass.
Examples of organic fillers that may be mentioned include polyamide powders (Nylon® Orgasol from Atochem), polyethylene powders, polymethyl methacrylate powders, polytetrafluoroethylene (Teflon) powders, acrylic acid copolymer powders (Polytrap from the company Dow Corning), lauroyl lysine, hollow polymer microspheres such as those of polyvinylidene chloride/acrylonitrile, for instance Expancel (Nobel Industrie), hexamethylene diisocyanate/trimethylol hexyllactone copolymer powder (Plastic Powder from Toshiki), silicone resin microbeads (for example Tospearl from Toshiba), synthetic or natural micronized waxes, metal soaps derived from organic carboxylic acids containing from 8 to 22 carbon atoms and preferably from 12 to 18 carbon atoms, for example zinc stearate, magnesium stearate, lithium stearate, zinc laurate or magnesium myristate, Polypore® L 200 (Chemdal Corporation), powders of crosslinked elastomeric organopolysiloxane coated with silicone resin, especially with silsesquioxane resin, as described, for example, in patent US 5 538 793, polyurethane powders, in particular powders of crosslinked polyurethane comprising a copolymer, said copolymer comprising trimethylol hexyllactone. It may in particular be a hexamethylene diisocyanate/trimethylol hexyllactone polymer. Such particles are especially commercially available, for example, under the name Plastic Powder D-400® or Plastic Powder D-800® from the company Toshiki, and mixtures thereof. It may also be cellulose powder, such as the product sold by Daito in the Cellulobeads range.

### Additional colorants

A composition according to the invention may also comprise at least one additional colorant, preferably in a proportion of at least 0.01% by weight relative to the total weight of the composition.

For obvious reasons, this amount is liable to vary significantly with regard to the intensity of the desired colour effect and of the colour intensity afforded by the colorants under consideration, and its adjustment clearly falls within the competence of those skilled in the art.

The additional colorants that are suitable for the invention are liposoluble.

For the purposes of the invention, the term *"liposoluble colorant"* is intended to mean any natural or synthetic, generally organic compound, which is soluble in an oily phase or in solvents that are miscible with a fatty substance, and which is capable of imparting colour.

As liposoluble dyes that are suitable for use in the invention, mention may be made especially of synthetic or natural liposoluble dyes, for instance DC Red 17, DC Red 21, DC Red 27, DC Green 6, DC Yellow 11, DC Violet 2, DC Orange 5, Sudan red, carotenes (β-carotene, lycopene), xanthophylls (capsanthin, capsorubin, lutein), palm oil, Sudan brown, quinoline yellow, annatto and curcumin.

## Moisturizing agent

A "moisturizing agent" is intended to mean, according to the present invention, any compound capable of penetrating into the stratum corneum and of keeping the latter moisturized.

The moisturizing agents that are usable according to the invention are notably chosen from polyols, urea and its derivatives, such as notably hydroxyalkyl urea, in particular hydroxyethyl urea such as the product sold under the trade name Hydrovance® by the company Akzo Nobel, hyaluronic acid, glycine, β-alanine, taurine, trimethyl glycine, and mixtures thereof.

For the purposes of the present invention, a "*polyol*" should be understood to be any organic molecule comprising at least two free hydroxyl groups.

According to one particular form, the polyol may be chosen from sugars such as trehalose, mannitol, xylitol, sorbitol, and mixtures thereof.

Preferably, a polyol according to the present invention is present in liquid form at ambient temperature.

A polyol that is suitable for use in the invention may be a compound of linear, branched or cyclic saturated or unsaturated alkyl type, bearing on the alkyl chain at least two -OH functions, in particular at least three -OH functions and more particularly at least four -OH functions.

The polyols that are advantageously suitable for formulating a composition according to the present invention are those in particular containing from 2 to 32 carbon atoms and preferably 3 to 16 carbon atoms.

Advantageously, the polyol may be chosen, for example, from ethylene glycol, pentaerythritol, trimethylolpropane, propylene glycol, 1,3-propanediol, butylene glycol, isoprene glycol, pentylene glycol, hexylene glycol, glycerol, polyglycerols, such as glycerol oligomers, for instance diglycerol, and polyethylene glycols, and mixtures thereof.

According to a preferred embodiment of the invention, said polyol is chosen from ethylene glycol, pentaerythritol, trimethylolpropane, propylene glycol, glycerol, polyglycerols, polyethylene glycols and mixtures thereof.

According to a particular mode, the composition of the invention may comprise at least propylene glycol and/or glycerol.

The moisturizing agent(s) are preferably present in the composition in a content ranging from 1% to 20%, better still from 3% to 15% by weight, preferably from 5% to 10% by weight, relative to the total weight of said composition.

## APPLICATIONS

According to one embodiment, a composition of the invention may advantageously be in the form of a composition for caring for the skin and/or keratin fibres, the body or the face, in particular the face.

According to another embodiment, a composition of the invention may advantageously be in the form of a composition for making up keratin materials, in particular the skin of the body or of the face, in particular of the face.

Thus, according to a sub-mode of this embodiment, a composition of the invention may advantageously be in the form of a makeup base composition.

A composition of the invention may advantageously be in the form of a foundation.

According to another sub-mode of this embodiment, a composition of the invention may advantageously be in the form of a composition for making up the skin and especially the face. It may thus be an eyeshadow or a face powder.

Such compositions are especially prepared according to the general knowledge of those skilled in the art.

Throughout the description, including the claims, the expression *"comprising a"* should be understood as being synonymous with *"comprising at least one",* unless otherwise specified.

The expressions *"between... and..."* and *"ranging from... to*..." should be understood as being inclusive of the limits, unless otherwise specified.

The invention is illustrated in greater detail by the examples and figures presented below. Unless otherwise indicated, the amounts shown are expressed as weight percentages.

## EXAMPLES

| INGREDIENTS | EXAMPLE 1 ACCORDING TO THE INVENTION | COMPARATIVE EXAMPLE 2 | COMPARATIVE EXAMPLE 3 | COMPARATIVE EXAMPLE 4 |
|---|---|---|---|---|
| Oleocraft LP-20 (Croda) (Polyamide-8) | 25% | 25% | - | - |
| Ethanol | 10% | | 10% | 10% |
| Isododecane | qs100% | qs100% | qs100% | qs100% |
| Silsoft 74 (Momentive Performance Materials) (trimethylsiloxysilicate at 75% in isododecane) | - | - | -25% A.M* | |
| Dow Corning ® 2-8178 geller (Nylon-611/dimethicone copolymer) | - | - | - | 25% |
| Yellow iron oxide coated with aluminium stearoyl glutamate<br>Red iron oxide coated with aluminium stearoyl glutamate | 15% | 15% | 15% | 15% |
| Black iron oxide coated with aluminium stearoyl glutamate<br>Titanium dioxide coated with aluminium stearoyl glutamate | | | | |
| *A. M: Active Material | | | | |

Procedure of Implementation Example 1 according to the invention: the ingredients are mixed for 1 hour at 90°C with a Rayneri mixer.

Procedure of Comparative Example 2: the ingredients are mixed for 1 hour at 90°C with a Rayneri mixer.

Procedure of Comparative Examples 3 and 4: the ingredients are mixed for 2 hours at ambient temperature with a Rayneri mixer.

Rheological measurements are carried out on these compositions. The measurements of the complex modulus of rigidity G* are carried out using a Haake RS600 rheometer on a product at rest, at 25°C. The following results are obtained:

| | EXAMPLE 1 ACCORDING TO THE INVENTION | COMPARATIVE EXAMPLE 2 | COMPARATIVE EXAMPLE 3 | COMPARATIVE EXAMPLE 4 |
|---|---|---|---|---|
| SPINDLE GEOMETRY | Plate/cone spindle<br>Ø 60 mm/2° | Plate/cone spindle<br>Ø 20 mm/1° | Plate/cone spindle<br>Ø 60 mm/2° | Plate/cone spindle<br>Ø 60 mm/2° |
| COMPLEX MODULUS OF RIGIDITY G* (stress ($\tau$) = 1 Pa) | 111 Pa | 36 700 Pa | < 1 Pa | 15 Pa |

The compositions are applied to the skin. After drying (tests carried out after 1 hour of drying at ambient temperature), the following results are obtained:

The deposit formed by the composition of the implementation example according to the invention has a good wear property on the skin and a high fluidity, thus making it easy to apply. It has, moreover, good resistance to water and to

soap. The resistance to water and to soap may be desired for making up the body, with the makeup result being resistant to showering, or making up the hands, with the makeup result withstanding washing of the hands.

The ethanol-free composition of Comparative Example 2 forms a gel which is more difficult to take up and to apply.

The composition with trimethylsiloxysilicate resin of Comparative Example 3 forms a deposit after application and drying on the skin which has a worse wear property than the deposit formed by the composition of the implementation example. The deposit formed by the composition of Comparative Example 3 crumbles when the deposit is rubbed with the finger, whereas the deposit formed by the composition of the implementation example remain intact.

The composition with Nylon-611/Dimethicone copolymer of Comparative Example 4 forms a deposit after application and drying on the skin which does not persist on the skin. When the deposit is rubbed with the finger, it is removed in the form of pilling.

## Claims

**1.** Fluid composition, **in the form of an anhydrous composition,** especially comprising a physiologically acceptable medium, especially for coating keratin materials, more particularly for making up and/or caring for keratin materials such as the skin, and containing

a) at least one ester-terminated poly(ester-amide) polymer; and
b) at least one volatile alkane; and
c) at least one monoalcohol comprising from 2 to 8 carbon atoms; and
d) a particulate phase comprising at least one pigment.

**2.** Composition according to Claim 1, wherein the ester-terminated poly(ester-amide) (ETPEA) polymer(s) are in the form of a resin prepared by reacting a diacid, a diamine, a polyol and a monoalcohol, in which:

(i) at least 50 equivalent % of said diacid comprises a polymerized fatty acid and
(ii) at least 50 equivalent % of said diamine comprises ethylenediamine.

**3.** Composition according to either one of Claims 1 and 2, wherein the diacid is a $C_{36}$ hydrogenated linoleic acid dimer.

**4.** Composition according to any one of Claims 1 to 3, wherein the diamine is ethylenediamine.

**5.** Composition according to any one of Claims 1 to 4, wherein the monoalcohol is stearyl alcohol.

**6.** Composition according to any one of Claims 1 to 5, wherein the polyol is neopentyl glycol.

**7.** Composition according to any one of Claims 1 to 6, **characterized in that** said ester-terminated poly(ester-amide) polymer is a polymer having the INCI name Polyamide-8.

**8.** Composition according to any one of Claims 1 to 7, **characterized in that** the ester-terminated poly(ester-amide) polymer(s) is (are) present in the composition in a content ranging from 1% to 35%, better still from 2% to 30% by weight, preferably from 3% to 25% by weight, relative to the total weight of said composition.

**9.** Composition according to any one of Claims 1 to 8, **characterized in that** the volatile alkane(s) is (are) a branched volatile alkane, preferably a $C_8$-$C_{16}$ branched volatile alkane, even more preferentially an isododecane.

**10.** Composition according to any one of Claims 1 to 9, **characterized in that** the volatile alkane(s) is (are) present in the composition in concentrations ranging from 35% to 95% by weight and more preferentially from 50% to 80% by weight relative to the total weight of the composition.

**11.** Composition according to one of Claims 1 to 10, **characterized in that** said monoalcohol c) is ethanol.

**12.** Composition according to one of Claims 1 to 11, **characterized in that** said composition comprises a ratio of ester-terminated poly(ester-amide) polymer(s) to the particulate phase greater than 0.5, preferably between 1 and 10.

**13.** Composition according to one of Claims 1 to 12, **characterized in that** said composition comprises a ratio of

alcohol(s) to alkane(s) of greater than 0.04 and preferably greater than 0.1.

**14.** Composition according to any one of Claims 1 to 13, comprising at least one pigment chosen from iron oxides and/or titanium dioxides, and more preferentially pigments of iron oxide and/or of titanium dioxide coated with at least one lipophilic or hydrophobic compound.

**15.** Composition according to one of Claims 1 to 14, **characterized in that** it comprises at least 5% by weight of pigments, more preferentially from 5% to 40% by weight of pigments, in particular from 10% to 30% by weight and preferably from 10% to 20% by weight of pigments, relative to the total weight of said composition.

**16.** Process for coating keratin materials, more particularly for making up and/or caring for keratin materials, such as the skin, **characterized in that** it comprises the application to the keratin materials of a composition as defined according to any one of Claims 1 to 15.

**Patentansprüche**

**1.** Fließfähige Zusammensetzung in Form einer wasserfreien Zusammensetzung, insbesondere umfassend ein physiologisch unbedenkliches Medium, insbesondere zum Beschichten von Keratinmaterialien, spezieller zum Schminken und/oder Pflegen von Keratinmaterialien wie der Haut, und enthaltend

a) mindestens ein Ester terminiertes Polyester-amid)-Polymer und
b) mindestens ein flüchtiges Alkan und
c) mindestens einen Monoalkohol mit 2 bis 8 Kohlenstoffatomen und
d) eine teilchenförmige Phase, die mindestens ein Pigment umfasst.

**2.** Zusammensetzung nach Anspruch 1, wobei das esterterminierte Poly(esteramid)(ETPEA)-Polymer bzw. die esterterminierten Poly(esteramid)(ETPEA)-Polymere in Form eines durch Umsetzung einer Disäure, eines Diamins, eines Polyols und eines Monoalkohols hergestellten Harzes vorliegen, wobei:

(i) mindestens 50 Äquivalent-% der Disäure eine polymerisierte Fettsäure umfassen und
(ii) mindestens 50 Äquivalent-% des Diamins Ethylendiamin umfassen.

**3.** Zusammensetzung nach einem der Ansprüche 1 und 2, wobei es sich bei der Disäure um ein hydriertes $C_{36}$-Linolensäuredimer handelt.

**4.** Zusammensetzung nach einem der Ansprüche 1 bis 3, wobei es sich bei dem Diamin um Ethylendiamin handelt.

**5.** Zusammensetzung nach einem der Ansprüche 1 bis 4, wobei es sich bei dem Monoalkohol um Stearylalkohol handelt.

**6.** Zusammensetzung nach einem der Ansprüche 1 bis 5, wobei es sich bei dem Polyol um Neopentylglykol handelt.

**7.** Zusammensetzung nach einem der Ansprüche 1 bis 6, **dadurch gekennzeichnet, dass** es sich bei dem esterterminierten Poly(esteramid)-Polymer um ein Polymer mit dem INCI-Namen Polyamide-8 handelt.

**8.** Zusammensetzung nach einem der Ansprüche 1 bis 7, **dadurch gekennzeichnet, dass** das esterterminierte Poly(esteramid)-Polymer bzw. die esterterminierten Poly(esteramid)-Polymere in der Zusammensetzung in einem Gehalt im Bereich von 1 bis 35 Gew.-%, noch besser von 2 bis 30 Gew.-%, vorzugsweise von 3 bis 25 Gew.-%, bezogen auf das Gesamtgewicht der Zusammensetzung, vorliegt bzw. vorliegen.

**9.** Zusammensetzung nach einem der Ansprüche 1 bis 8, **dadurch gekennzeichnet, dass** es sich bei dem flüchtigen Alkan bzw. den flüchtigen Alkanen um ein verzweigtes flüchtiges Alkan, vorzugsweise ein verzweigtes flüchtiges $C_8$-$C_{16}$-Alkan, noch weiter bevorzugt ein Isododecan, handelt.

**10.** Zusammensetzung nach einem der Ansprüche 1 bis 9, **dadurch gekennzeichnet, dass** das flüchtige Alkan bzw. die flüchtigen Alkane in der Zusammensetzung in Konzentrationen im Bereich von 35 bis 95 Gew.-% und weiter bevorzugt von 50 bis 80 Gew.-%, bezogen auf das Gesamtgewicht der Zusammensetzung, vorliegt bzw. vorliegen.

11. Zusammensetzung nach einem der Ansprüche 1 bis 10, **dadurch gekennzeichnet, dass** es sich bei dem Monoalkohol c) und Ethanol handelt.

12. Zusammensetzung nach einem der Ansprüche 1 bis 11, **dadurch gekennzeichnet, dass** die Zusammensetzung ein Verhältnis von esterterminiertem Polyesteramid)(ETPEA)-Polymer bzw. esterterminierten Poly(esteramid) (ETPEA)-Polymeren zu der teilchenförmigen Phase von mehr als 0,5, vorzugsweise zwischen 1 und 10, umfasst.

13. Zusammensetzung nach einem der Ansprüche 1 bis 12, **dadurch gekennzeichnet, dass** die Zusammensetzung ein Verhältnis von Alkohol bzw. Alkoholen zu Alkan bzw. Alkanen von mehr als 0,04 und vorzugsweise mehr als 0,1 umfasst.

14. Zusammensetzung nach einem der Ansprüche 1 bis 13, umfassend mindestens ein Pigment, das aus Eisenoxiden und/oder Titandioxiden und weiter bevorzugt Pigmenten von Eisenoxid und/oder Titandioxid, die mit mindestens einer lipophilen oder hydrophoben Verbindung beschichtet sind, ausgewählt ist.

15. Zusammensetzung nach einem der Ansprüche 1 bis 14, **dadurch gekennzeichnet, dass** sie mindestens 5 Gew.-% Pigmente, weiter bevorzugt 5 bis 40 Gew.-% Pigmente, insbesondere 10 bis 30 Gew.-% und vorzugsweise 10 bis 20 Gew.-% Pigmente, bezogen auf das Gesamtgewicht der Zusammensetzung, umfasst.

16. Verfahren zum Beschichten von Keratinmaterialien, spezieller zum Schminken und/oder Pflegen von Keratinmaterialien, wie der Haut, **dadurch gekennzeichnet, dass** es das Aufbringen einer Zusammensetzung gemäß einem der Ansprüche 1 bis 15 auf die Keratinmaterialien umfasst.

## Revendications

1. Composition fluide, sous la forme d'une composition anhydre, notamment comprenant un milieu physiologiquement acceptable, notamment pour le revêtement de matières kératiniques, plus particulièrement pour le maquillage et/ou le soin des matières kératiniques telles que la peau, et contenant

   a) au moins un polymère de type poly(ester-amide) à terminaison ester, et
   b) au moins un alcane volatil, et
   c) au moins un monoalcool comprenant de 2 à 8 atomes de carbone ; et
   d) une phase particulaire comprenant au moins un pigment.

2. Composition selon la revendication 1, où le ou les polymère(s) de type poly(ester-amide) à terminaison ester (ETPEA) sont sous la forme d'une résine préparée en faisant réagir un diacide, une diamine, un polyol et un monoalcool, dans laquelle :

   (i) au moins 50 équivalent % dudit diacide comprennent un acide gras polymérisé et
   (ii) au moins 50 équivalent % de ladite diamine comprennent de l'éthylènediamine.

3. Composition selon l'une ou l'autre des revendications 1 et 2, où le diacide est un dimère de l'acide linoléique hydrogéné en $C_{36}$.

4. Composition selon l'une quelconque des revendications 1 à 3, où la diamine est l'éthylènediamine.

5. Composition selon l'une quelconque des revendications 1 à 4, où le monoalcool est l'alcool stéarylique.

6. Composition selon l'une quelconque des revendications 1 à 5, où le polyol est le néopentylglycol.

7. Composition selon l'une quelconque des revendications 1 à 6, **caractérisée en ce que** ledit polymère de type poly(ester-amide) à terminaison ester est un polymère ayant le nom INCI Polyamide-8.

8. Composition selon l'une quelconque des revendications 1 à 7 **caractérisée en ce que** le ou les polymère (s) de type poly(ester-amide) à terminaison ester est (sont) présent(s) dans la composition en une teneur allant de 1 % à 35 %, mieux de 2 % à 30 % en poids, de préférence de 3 % à 25 % en poids, par rapport au poids total de ladite composition.

9. Composition selon l'une quelconque des revendications 1 à 8 **caractérisée en ce que** le ou les alcane(s) volatil(s) est (sont) un alcane volatil ramifié, de préférence un alcane volatil ramifié en $C_8$-$C_{16}$, encore plus préférentiellement un isododécane.

10. Composition selon l'une quelconque des revendications 1 à 9 **caractérisée en ce que** le ou les alcane(s) volatil(s) est (sont) présent(s) dans la composition en des concentrations allant de 35 % à 95 % en poids et plus préférentiellement de 50 % à 80 % en poids par rapport au poids total de la composition.

11. Composition selon l'une des revendications 1 à 10, **caractérisée en ce que** ledit monoalcool c) est l'éthanol.

12. Composition selon l'une des revendications 1 à 11 **caractérisée en ce que** ladite composition comprend un rapport entre le ou les polymère(s) de type poly(ester-amide) à terminaison ester sur la phase particulaire supérieur à 0,5, de préférence compris entre 1 et 10.

13. Composition selon l'une des revendications 1 à 12, **caractérisée en ce que** ladite composition comprend un rapport d'alcool(s) sur alcane(s) supérieur à 0,04 et de préférence supérieur à 0,1.

14. Composition selon l'une quelconque des revendications 1 à 13, comprenant au moins un pigment choisi parmi les oxydes de fer et/ou les dioxydes de titane, et plus préférentiellement, des pigments d'oxyde de fer et/ou de dioxyde de titane revêtus par au moins un composé lipophile ou hydrophobe.

15. Composition selon l'une des revendications 1 à 14, **caractérisée en ce qu'**elle comprend au moins 5 % en poids de pigments, plus préférentiellement de 5 % à 40 % en poids de pigments, en particulier de 10 % à 30 % en poids et de préférence de 10 % à 20 % en poids de pigments, par rapport au poids total de ladite composition.

16. Procédé pour le revêtement de matières kératiniques, plus particulièrement pour le maquillage et/ou le soin de matières kératiniques telles que la peau, **caractérisé en ce qu'**il comprend l'application sur les matières kératiniques d'une composition telle que définie selon l'une quelconque des revendications 1 à 15.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20050197479 A
- WO 2018115771 A
- US 6552160 B
- US 3157681 A
- WO 2007068371 A
- WO 2008155059 A
- US 4578266 A
- JP H07196946 B
- US 5725882 A
- US 5209924 A
- US 4972037 A
- US 4981903 A
- US 4981902 A
- US 5468477 A
- US 5219560 A
- EP 0388582 A
- US 5246694 A
- EP 0486135 A
- JP H0586984 B
- FR 2679771
- EP 1184426 A
- US 5538793 A


### Non-patent literature cited in the description


- Naval Stores--Production, Chemistry and Utilization. Pulp. Chem. Assoc. Inc, 1989
- **H. P. FIEDLER.** Dictionary For Auxiliaries For Pharmacy, Cosmetics And Related Fields. Cantor Aulendorf, 1989
- **VAN DE HULST, H.C.** Light Scattering by Small Particles. Wiley, 1957
- **WITUCKI.** A silane primer, Chemistry and applications of alkoxy silanes. *Journal of Coatings Technology,* 1993, vol. 65 (822), 57-60
- **C.M. HANSEN.** The three-dimensional solubility parameters. *J. Paint Technol.,* 1967, vol. 39, 105